# EUROPEAN PATENT SPECIFICATION

(11) **EP 1 325 748 B1**
(45) Date of publication and mention of the grant of the patent: **12.07.2006**
(21) Application number: 01930119.1
(22) Date of filing: 14.05.2001
(51) Int. Cl.: A61K 31/7048, A61K 31/352, A23L 1/30, A61P 35/00, C07H 17/065, C07D 311/62

(54) **CYANIDIN MIXTURE FOR THE TREATMENT OF LARGE INTESTINE CANCER**
CYANIDINMISCHUNG ZUR BEHANDLUNG VON DICKDARMKREBS
MEDICAMENTS PREVENTIFS OU THERAPEUTIQUES CONTRE LE CANCER AYANT COMME PRINCIPE ACTIF DES COMPOSES DE CYANIDINE

(30) Priority: 11.08.2000 JP 2000245165
(43) Date of publication of application: 09.07.2003
(73) Proprietor: SAN-EI GEN F.F.I., INC., Toyonaka-shi, Osaka 561-8588 (JP)
(72) Inventor: SHIRAI, Tomoyuki, Nagoya-shi, Aichi 458-0003 (JP); IMAIDA, Katsumi, Nagoya-shi, Aichi 465-0062 (JP); HAGIWARA, Akihiro, Kani-shi, Gifu 509-0261 (JP); TAMANO, Seiko, Ichinomiya-shi, Aichi 491-0082 (JP); NAKAMURA, Mikio, San-Ei Gen F.F.I., Inc., Toyonaka-shi, Osaka 561-8588 (JP); KODA, Takatoshi, San-Ei Gen F.F.I., Inc., Toyonaka-shi, Osaka 561-8588 (JP)
(74) Representative: Sama, Daniele
(86) International application number: PCT/JP2001/004001
(87) International publication number: WO 2002/013836

(56) References cited:
- WO-A-97/41137
- JP-A- 2000 178 295
- DATABASE WPI Section Ch, Week 199414 Derwent Publications Ltd., London, GB; Class D13, AN 1994-110003 XP002305660 & CN 1 073 461 A (UNIV QINGDAO OCEAN) 23 June 1993 (1993-06-23)
- HARBORNE J B ET AL: "MALONATED CYANIDIN 3-GLUCOSIDES IN ZEA MAYS AND OTHER GRASSES" PHYTOCHEMISTRY, PERGAMON PRESS, GB, vol. 26, no. 8, 1987, pages 2417-2418, XP001071231 ISSN: 0031-9422
- HAGIWARA A ET AL: "Pronounced inhibition by a natural anthocyanin, purple corn color, of 2-amino-1-methyl-6-phenylimidazo[4,5-b]pyr idine (PhIP)-associated colorectal carcinogenesis in male F344 rats pretreated with 1,2-dimethylhydrazine." CANCER LETTERS. 28 SEP 2001, vol. 171, no. 1, 28 September 2001 (2001-09-28), pages 17-25, XP002305659 ISSN: 0304-3835
- KAMEI H. ET AL: 'Suppression of tumor cell growth by anthocyanins in vitro' CANCER INVESTIGATION vol. 13, no. 6, 1995, pages 590 - 594, XP000995356

## Description

### Technical Field

The present invention relates to the use of a mixture of two cyanidins as described in claim 1 for the manufacture of a medicament or a health food for the treatment of large intestine cancer.

### Background Art

Anthocyanin pigments having a wide distribution in plant kingdom have been utilized as coloring agents and known as having an antioxidation property as a physiological activity (Japanese Unexamined Patent Publication No. HEI 6-271850). In recent years, it has been pointed out that the pigments inhibit in vitro mutagenicity of a heterocyclic amine, an environmental carcinogen (Jpn. Soc. for Cancer Prevention, 7^{th}, July 14-15, 2000).

WO97/41137 discloses the use of anthocyanidin and its derivatives for the treatment of neoplastic disorders. As examples compounds of formula III and IV were mentioned. However it does not describe, nor does it suggest the specific combination of present cyanidins for the treatment of the specific large intestine cancer.

JP2000178295 describes compound of formula III for the treatment of duodenal cancer, that is to say a cancer in the first portion of the small intestin. It mentions purple corn among a large list of possible source of compound of formula III. However it does not mention the treatment of large intestine cancer.

### Disclosure of Invention

As a result of eager researches on the functions of the anthocyanin pigments, the inventors of the present invention have found that a particular cyanidin mixture has a carcinostatic effect against large intestine cancer and thus achieved the present invention.

According to the present invention, provided are a preventive or therapeutic agent for for large intestine cancer containing a cyanidin mixtures as described in claim 1.

The general formula (I):

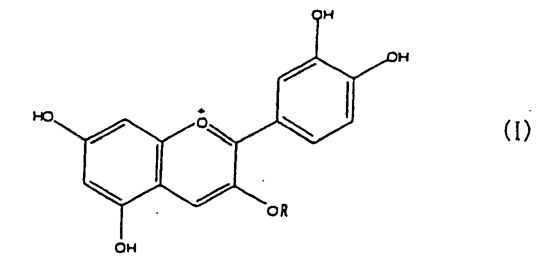

### Brief Description of Drawings

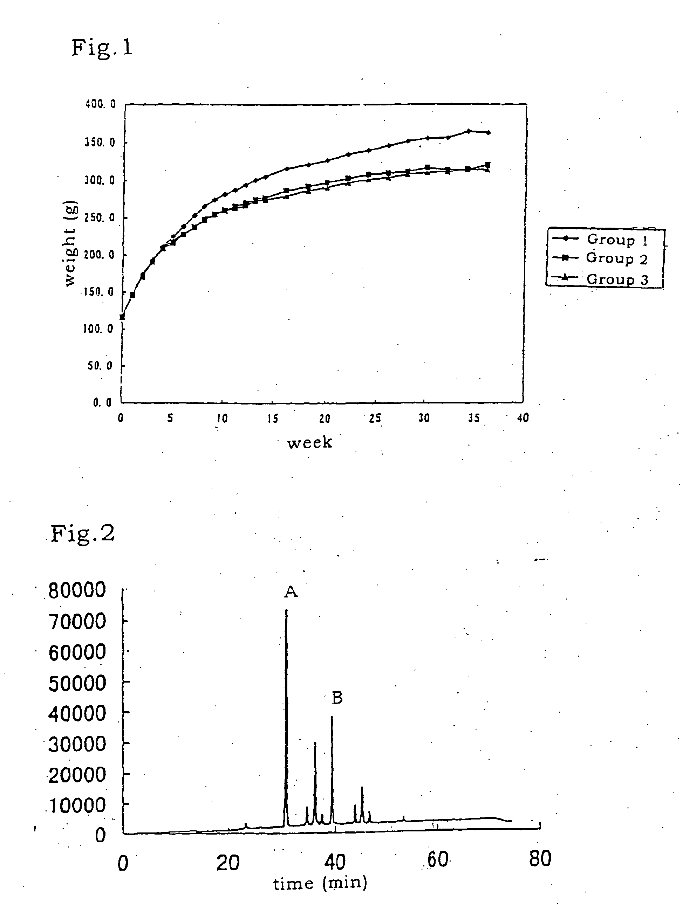

Fig. 1 illustrates increase in weight of rats administrated with various substances after treatment with DMH (1,2-dimethylhydradine dihydrochloride) which is used as an initiator of large intestine cancer. Rats in group 1 are administrated with a basic feed, those in group 2 are administrated with a carcinogenesis promoting substance PhIP (2-amino-1-methyl-6-phenylimidazo[4,5-b]pyridine) and those in group 3 are administrated with a mixture of PhIP (200 ppm) and a purple corn pigment (5 %).
Fig. 2 shows the results of HPLC analysis of a purple corn pigment solution prepared in Example 1. It shows individual peaks of the compound represented by the formula (III) (peak A) and the compound represented by the formula (IV) (peak B).

### Best Mode for Carrying Out the Invention

The cyanidin compound represented by the general formula (I) includes 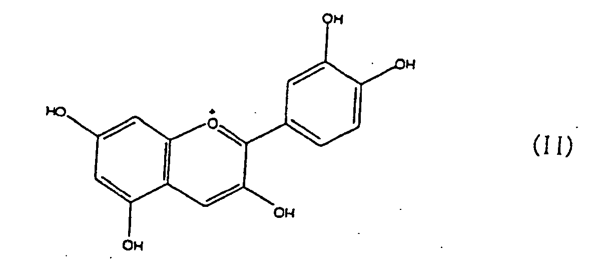 cyanidin represented by the formula (II); 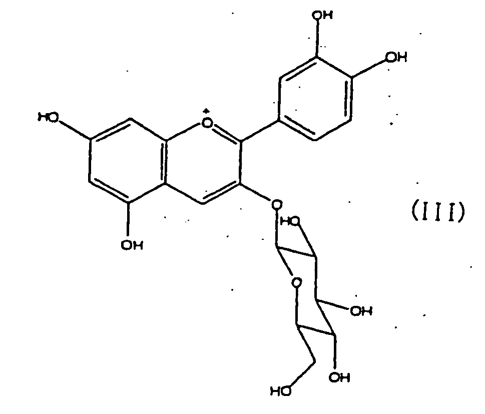 3-*O*-(β-D-glucopyranosyl)cyanidin represented by the formula (III); and 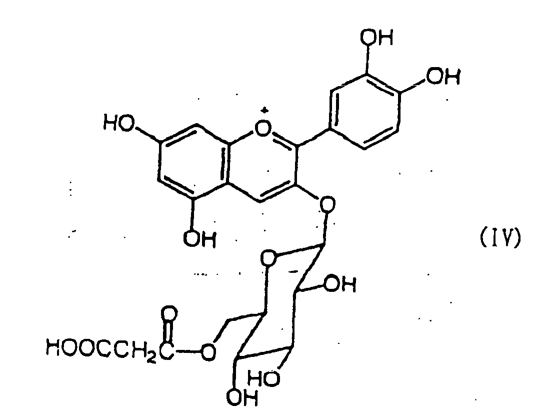 3-*O*-(6-malonyl- β-D-glucopyranosyl)cyanidin represented by the formula (IV).

Among these cyanidin compounds, the compounds of the formulae (III) and (IV) may be synthesized by a method known in the art, but may be generally obtained by extraction from a plant containing them or a cultured cell of the plant. The compound of the formula (II) can be easily obtained by hydrolyzing the compound of the formula (III) or (IV).

As the plant, one or more kinds of plants may be selected from the group consisted of purple corn, red cabbage, berries such as strawberry, boysenberry, raspberry, cranberry, blackberry and blueberry, grape and hibiscus. Any portion of the plants, such as stems, leaves, roots, petals, fruits and seeds, may be used. For example, seeds of purple corn, leaves of red cabbage, fruits of berries, pericarps of grapes, petals of hibiscus are preferably used.

For the extraction, the plant may be used directly or cut or crushed into an appropriate size. Alternatively, dried plants in these various shapes may be used. However, for a sufficient extraction of the cyanidin compound from the plant, it is preferred to use the plant cut into 1-2 cm, for example, without drying.

The method of extracting the cyanidin compound from the plant is not particularly limited, but for example, water or hydrated alcohol (water containing 40 or less % by volume, e.g., about 25 % by volume of alcohol) is commonly used as an extraction solvent. However, the extraction may be performed by first using a lower alcohol such as methanol and ethanol and then using water.

Temperature for the extraction may be room temperature or a temperature higher than room temperature. Period for the extraction varies depending on the temperature and is not particularly limited, but may be 4-12 hours in general. The extraction may be carried out effectively under an acidic condition of pH about 1-4 by using an inorganic acid such as sulfuric acid or an organic acid such as citrate.

After separating a solid portion, an extract solution thus obtained is appropriately concentrated to be a water soluble extract. This is subjected to an isolation and purification treatment by suitably combining common treatment methods such as resin adsorption and filtration using a nonionic adsorptive resin or an ion exchange resin. Thus, the compounds of formulae (III) and (IV) are obtained.

The usable nonionic adsorptive resins include, for example, Duolite S-861, Duolite S-862, Duolite S-863 and Duolite S-866 of styrene series (manufactured by Diamond Shamrock U.S.A.); Sepabeads SP70, Sepabeads SP700 and Sepabeads SP825 of aromatic series (manufactured by Mitsubishi Chemical Corporation); Diaion HP10, Diaion HP20, Diaion HP21, Diaion HP40 and Diaion HP50 (manufactured by Mitsubishi Chemical Corporation); and Amberlite XAD-4, Amberlite XAD-7 and Amberlite XAD-2000 (manufactured by Organo Corporation).

Also, usable cation exchange resins include Diaion SK 1B, Diaion SK 102, Diaion SK 116, Diaion PK 208, Diaion WK 10 and Diaion WK 20 (manufactured by Mitsubishi Chemical Corporation) and usable anion exchange resins include Diaion SA 10A, Diaion SA 12A, Diaion SA 20A, Diaion PA 306, Diaion WA 10 and Diaion WA 20 (manufactured by Mitsubishi Chemical Corporation).

The filtration may be ultrafiltration or reverse osmosis using various functional polymer membranes.

The inventors of the present invention have realized that the cyanidin compound obtained by the above method shows a carcinostatic effect against cancer, in particular large intestine cancer.

Accordingly, the present invention provides a preventive or therapeutic agent for large intestine cancer containing the cyanidin mixtures as described in claim 1 or a water soluble extract from a plant containing the said mixture.

The preventive or therapeutic agent is manufactured by a conventional method using a solid or liquid excipient known in the art. Dosage form of the agent may be dispersion, tablet, emulsion, capsule, granule, chewable tablet, solution, syrup and the like. These agents may be obtained by a conventional method using various excipients.

The solid excipients include, for example, lactose, sucrose, glucose, corn starch, gelatin, starch, dextrin, calcium phosphate, magnesium oxide, dried aluminum hydroxide, sodium bicarbonate and the like. The liquid excipients include, for example, water, glycerin, propylene glycol, simple syrup, ethanol, fat oil, ethylene glycol, polyethylene glycol, sorbitol and the like.

The above-described agent may contain conventional additives such as a buffer, a stabilizer, a sweetener, a preservative, a flavoring agent and a coloring agent, if desired.

The health food as used the present invention signifies a food positively intended to health care, health maintenance and health enhancement compared with the common food. The food added with the cyanidin compound or the water soluble plant extract containing the compound according to the present invention can be the health food expected to exhibit effects of preventing or treating large intestine cancer. For example, the health food can be obtained by mixing or spraying the cyanidin compound of the invention with or into an intermediate product under processing or a final product, for example, solid, liquid or semisolid products as well as processed or half-processed products thereof such as starch, wheat flour, sugar and syrup which can be used food as they are ; bread, noodles, sweets including candies and cookies, other solid products ; flavor enhancers such as dressings and sauces ; liquid products such as beverage, nutritional drink and soup ; semisolid products such as jerry.

In the case of medical use, the compound of the formula (I) may be administrated as an active ingredient to an adult of 60 kg in an amount of 0.5 to 50 mg a day, preferably 30 to 50 mg a day. In the case of health food application, the compound of the formula (I) may be added to the food in such an amount that the compound does not influence a taste and an appearance of the food, e.g., an amount of 10 to 30 mg, which is greater than 1 to 10 mg, i.e., the amount of the compound when added as the coloring agent.

However, the dose amount of the compound may vary depending on various factors such as health conditions of a person who will take the cyanidin compound, an application method and a combination with other agents.

The cyanidin compound represented by the general formula (I) is originally contained in various natural plants and has been used as a food additive. Thus, the cyanidin compound can safely and effectively be utilized as a substance for preventing or treating cancer, and economically manufactured.

### Examples

Hereinafter, the present invention will be detailed with reference to examples, but the invention is not limited thereto.

### Example 1:

To a mixture solution of 16 L of water, 4 L of ethanol and 90 g of concentrated sulfuric acid (pH 2.3), 2 kg of dried purple corn (a mixture of a cob of 23 % and kernels of 77 %, the cob was cut into the size of 1-2 cm) were added and allowed to stand overnight at room temperature to extract a red pigment. After the extraction, the pigment was subjected to solid-liquid separation using a metal gauze of 60 mesh. This solution was added with a 1 % filter aid (Radiolite #500 manufactured by Showa Kagaku Kogyo Co., Ltd.) and filtrated to obtain a water soluble extract solution of the purple corn pigment of about 16 L.

The thus obtained extract was subjected to adsorption using a synthetic adsorptive resin Amberlite XAD-7 (1.5 L, manufactured by Organo Corporation). After the resin was sufficiently washed with 5 L of water, hydrated ethanol of 0-80 % concentration gradient was used to collect red color fractions through visual observation. The elution of 4.8 L was obtained.

The elution was treated using an ultrafiltration membrane (AHP-2013 membrane: manufactured by Asahi Kasei Corporation, fractional molecular weight of 50,000) under 3 kg/cm² at 20°C. Then, dilute sulfuric acid was added to the filtrate to adjust pH to 2.0 and 5 L of water was added. Then, a reverse osmosis membrane (NTR-7250 membrane: manufactured by Nitto Denko Co., Ltd., fractional molecular weight of about 3000) was used to remove contaminants through permeation to give a 1 L residual solution comprising purified pigment components.

Then, the solution was concentrated under reduced pressure and a 80 g purified pigment solution having a color valency of E10% 1 cm = 200 (calculated by measuring absorbency at a maximum absorption wavelength (around 510 nm) in a visible region of the pigment-containing solution and converting it into absorbency of a 10 w/v% solution) was obtained.

In the pigment solution, 3.4 wt% of the compound of the formula (III) and 1.7 wt% of the compound of the formula (IV), which are active ingredients for the carcinostatic effect against large intestine cancer according to the present invention, were contained.

To 80 g of the pigment solution, 130g of water, 40 g of ethanol and 10 g of citric acid (crystals) were added to prepare 260 g of purple corn pigment solution having a color valency of E10% 1 cm=60.

### Example 2:

Using DMH (1,2-dimethylhydradinedihydrochloride, manufactured by Tokyo Kasei Kogyo Co., Ltd., Lot. FIH01-UR) as an initiator of large intestine cancer, the carcinostatic effect of a purple corn pigment (manufactured by San-Ei gen F.F.I., Inc. Lot. 990519, containing 10.8 % of the compound of the formula (III) and 6.5 % of the compound of the formula (IV), hereinafter referred to as PCC) was examined against the carcinogenesis promoting effect of PhIP (2-amino-1-methyl-6-phenylimidazo [4,5-b] pyridine (Toronto Research Chemical Inc., Lot. 5-YCX-55-2).

F344/DuCrj male rats (Charles River Japan Inc.) were used as test animals. The rats were subjected to a subcutaneous administration of DMH of 20 mg/kg once a week, for 4 weeks. They were divided into a group 1 fed with a basic feed (labo MR stock, manufactured by Nihon Nosan Kogyo KK), a group 2 fed with PhIP (200 ppm) only, and a group 3 fed with a mixture of PhIP (200 ppm) and PCC (5%). The rats (20 per group) were allowed to take the feed freely for 32 weeks.

### a) Survival rate and clinical manifestation

The rats survived until the end of the experiment except one in the group 2 died during the experiment.

In the group 3, the rats showed coloration of fur and black-colored excrement that seem to be derived from the administration of PCC.

### b) Weight and intake amount of feed

With an electronic balance (LC2200, manufactured by Sartorius), the weight of each rat was measured once a week until 14^{th} week after initiation of the experiment, and thereafter measured once every two weeks. The feed intake amount of each rat was also measured once a week until 14^{th} week, and thereafter measured once every other week in the same manner as the weight measurement, except that the feed intake amount for two days was measured.

During the DMH treatment, the rats of every group weighed similarly in the range of about 116 to 210 g. However, during the administration of the test substances, the weight of the rats of the groups 2 and 3 increased to a similar extent from about 230 g to 315 g, but the increase was significantly smaller than that of the group 1 (increased to about 360 g). In every group, no significant difference was observed in the feed intake amount (Fig. 1).

In the group 3, an average intake amount of PCC obtained from an administration concentration, an average weight and an average feed intake amount was 3744.38 mg/kg/day. An average intake amount of PhIP was 11.98 mg/kg/day in the group 2 and 14.98 mg/kg/day in the group 3.

### c) Intake amount of water

With an electronic balance (LC2200, manufactured by Sartorius), an intake amount of water for two days was measured once a week until 14^{th} week, and thereafter measured once every other week.

During the DMH treatment, the rats showed similar water intake amount in every group. In the 5^{th} and 6^{th} weeks during the administration of the test substances, the water intake amount of the rats in the group 3 (about 24 g/animal/day) increased as compared with that in the group 1 (about 20 g/animal/day) and that in the group 2 (about 19 g/animal/day). After these weeks, the values were almost the same as those in the other groups.

### d) Macroscopic pathologic examination

The rats alive at the end of the experiment were fasted and then bled for euthanasia under deep anesthesia. They were dissected together with the dead rat of the group 2 to macroscopically examine tumorigenesis in various organs in the thoracic cavity and the abdominal cavity. Table 1 shows the results.

**Table 1**

| Group | Number of rats having tumor | Number of tumors | Number of tumors/number of rats having tumor |
|---|---|---|---|
| Group 1 | 4 | 5 | 1.25 |
| Group 2 | 17 | 48 | 2.82 |
| Group 3 | 8 | 9 | 1.13 |

In every rat, tumorigenesis was not observed in other organs than the large intestine (cecum, colon, rectum). The tumor generated in the large intestine was recognized as cancer by the macroscopic pathologic examination.

### e) Conclusion

As shown in Table 1, the rats in the group administrated with PCC showed the number of tumors per animal similar to that of the rats in the group administrated with the basic feed. It was recognized that PCC inhibits the carcinogenesis promoting effect by PhIP without causing any bad influences such as increase in weight.

### Example 3:

The purple corn pigment solution obtained in Example 1 was mixed with materials according to the recipe below and the mixture was filtrated. This was then filled in a 250 ml bottle and sterilized at 90°C for 30 minutes to prepare a berry flavored soft drink containing fruit juice.

| | |
|---|---|
| Sugar | 7.50 |
| High fructose corn syrup (Brix 75°) | 5.00 |
| Citrate (crystals) | 0.20 |
| 1/5 concentrated clear apple juice | 2.00 |
| purple corn pigment solution | 0.10 |
| Berry flavor | 0.05 |
| Water | 85.15 |
| Total | 100.00 kg |

With the soft drink of 500 ml, the intake amounts of the compounds of the formulae (III) and (IV) were 4.65 mg and 2.33 mg, respectively.

### Example 4:

With the purple corn pigment solution obtained in Example 1, hard candies were prepared under the following recipe.

The pigment solution was added together with citrate to a mixture obtained by heating and melting granulated sugar, starch syrup and water at 150°C. This was molded to obtain the candies.

| | |
|---|---|
| Granulated sugar | 65.0 |
| Starch syrup | 50.0 |
| Water | 10.0 |
| Citrate (crystals) | 0.5 |
| purple corn pigment solution | 0.1 |
| Total | 100.00 kg |

### Referential example 1:

Components of the purple corn pigment solution prepared in Example 1 were analyzed by HPLC. The solution of 0.1 g was mixed in 100 ml of distilled water and 20 µ1 of the mixture was injected in a L-column ODS (diameter 4.6 X 250 mm, manufactured by Chemicals Evaluation and Research Institute, Japan). As a mobile phase, 0.5 % trifluoroacetic acid and acetonitrile were used. Linear gradient was provided by using acetonitrile of 10-15 % for 10 minutes, 15-30 % for 50 minutes, 30-100 % for 5 minutes, and 100 % for 5 minutes.

Intensity at 510 nm was detected under a flow rate of 1.0 ml/min at 40°C.

Thus, the results shown in Fig. 2 were obtained. The compound (A) of the formula (III) and the compound (B) of the formula (IV) showed peaks, respectively (Fig. 2).

According to the present invention, the cyanidin mixture as described in claim 1 contained naturally in various plants can be utilized as a agent safely and effectively preventing and treating large intestine cancer.

## Claims

1. Use of a mixture of 3-O-(β-D-glucopyranosyl)cyanidin represented by the formula (III): 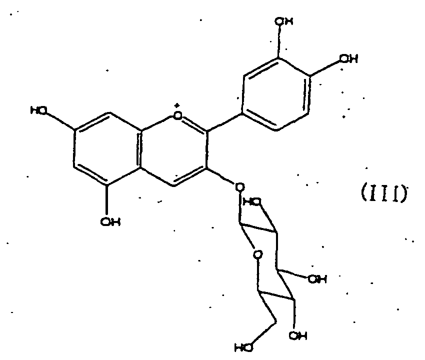 and 3-O-(6-malonyl-β-D-glucopyranosyl)cyanidin represented by the formula (IV): 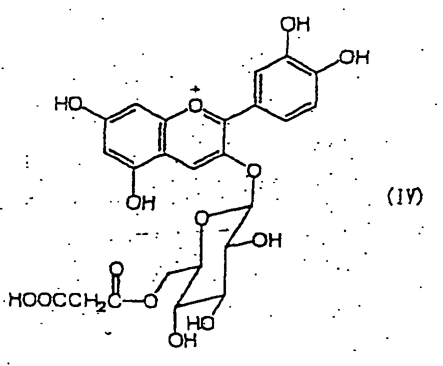 for the manufacture of a medicament or a food composition for the treatment or prevention of large intestine cancer.

2. Use according to claim 1, wherein the mixture is used as a water soluble extract from a plant.

3. Use according to claim 2, wherein the plant is purple corn.

4. Use according to any one of claims 1 to 3, the mixture is for the manufacture of a food composition.

## Patentansprüche

1. Anwendung einer Mischung aus 3-0-(β-D-Glucopyranosyl)cyanidin, dargestellt durch die Formel (III): 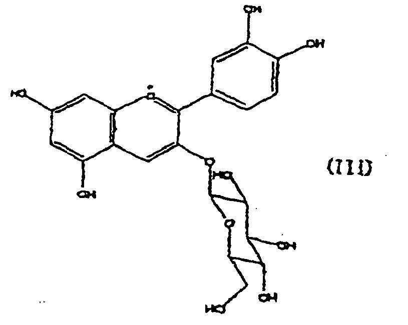 und 3-0-(6-Malonyl-β-D-Glucopyranosyl)cyanidin, dargestellt durch die Formel (IV): 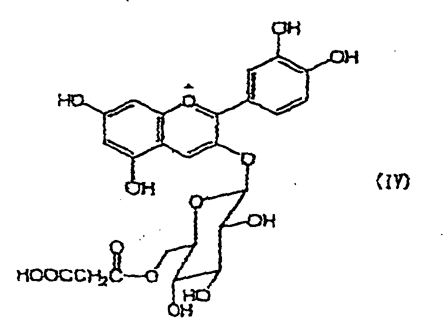 zur Herstellung eines Medikaments oder einer Nahrungsmittelzusammensetzung zur Behanttlung oder Vorbeugung von Dickdarmkrebs.

2. Anwendung nach Anspruch 1, worin die Mischung als ein wasserlösliches Extrakt einer Pflanze benutzt wird.

3. Anwendung nach Anspruch 2, worin die Pflanze lilla Mais ist.

4. Anwendung nach irgendeinem der Ansprüche 1 bis 3, wobei die Mischung für die Herstellung einer Nahrungsmittelzusammensetzung vorgesehen ist.

## Revendications

1. Utilisation d'un mélange de 3-O-(β-D-glucopyranosyl)cyanidine représenté par la formule (III) : 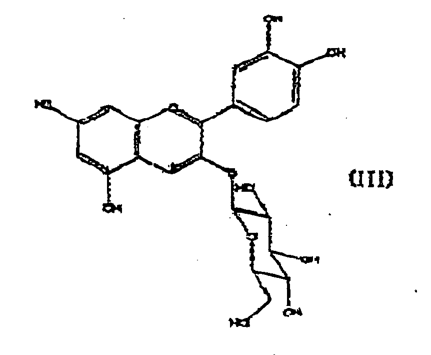 et de 3-O-(6-malonyl-β-D-glucopyranosyl)cyanidine représenté par la formule (IV) : 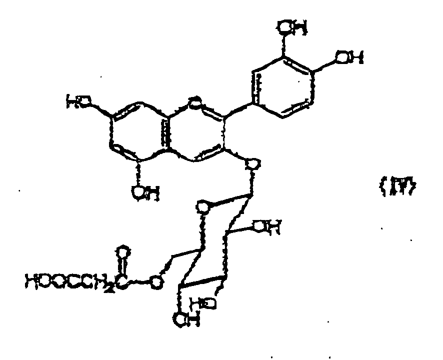 pour la fabrication d'un médicament ou d'une composition alimentaire destiné au traitement ou à la prévention d'un cancer du gros intestin.

2. Utilisation selon la revendication 1, dans laquelle le mélange est utilisé sous la forme d'un extrait hydrosoluble d'une plante.

3. Utilisation selon la revendication 2, dans laquelle la plante est le maïs mauve.

4. Utilisation selon l'une quelconque des revendications 1 à 3, dans laquelle le mélange est destiné à la fabrication d'une composition alimentaire.
